# EUROPEAN PATENT APPLICATION

(11) **EP 4 545 111 A1**
(43) Date of publication of application: **30.04.2025**
(21) Application number: 23206255.4
(22) Date of filing: 26.10.2023
(51) Int. Cl.: A61M 1/00

(54) **NEGATIVE PRESSURE WOUND THERAPY**

(71) Applicant: Mölnlycke Health Care AB, 402 52 Göteborg (SE)
(72) Inventor: UVEBORN, Johan, 436 40 Askim (SE); BENGTSSON, Erik, 431 41 Mölndal (SE); VANNAS, Alexander, 421 51 Göteborg (SE); DOLANGE, Guillaume, 412 78 Mölndal (SE)
(74) Representative: Zacco Sweden AB

(57) **Abstract**

An apparatus and related aspects for providing negative pressure to a wound site are disclosed. The apparatus comprises a source of negative pressure configured to provide negative pressure via a first fluid flow path from an inlet of the source of negative pressure to a wound site, and an electronically controllable valve configured to release negative pressure from the wound site via a second fluid flow path. The apparatus further comprises a pressure sensor configured to measure a pressure level in the second fluid flow path, and control circuitry operatively connected to the source of negative pressure, the electronically controllable valve, and the pressure sensor. The control circuitry is configured to open the electronically controllable valve, and in response to a pressure change rate being above a pressure change rate value, output a signal indicative of a blockage in the second fluid flow path. The control circuitry is further configured to in response to the pressure change rate being below the pressure change rate value, close the electronically controllable valve and activate the source of negative pressure, and in response to the pressure level, as measured by the pressure sensor, being non-responsive to a change in negative pressure generated while the source of negative pressure is active, output a signal indicative of a blockage in the first fluid flow path.

## Description

### TECHNICAL FIELD

The herein disclosed embodiments generally relate to negative pressure wound therapy (NPWT). In particular, the herein disclosed embodiments relate to apparatuses for providing negative pressure to a wound site, methods for controlling an apparatus for providing negative pressure to a wound site, and thereto related computer program products and computer-readable storage media.

### BACKGROUND

Negative pressure wound therapy (NPWT) is a technique that promotes healing of e.g. surgical, acute and chronic wounds by the application of a sub-atmospheric pressure ("negative pressure") to the wound site, using a negative pressure pump. Wound healing is achieved by applying a negative pressure, such as "vacuum" through a dressing or a cover applied onto the wound. Excess wound exudate is thereby drawn out, which increases the blood flow to the area, and promotes the formation of granulation tissue. The NPWT technique also permits less outside disturbance of the wound and transports excess fluids away from the wound site.

The negative pressure applied to the wound site may need to be properly managed to maintain or increase the effectiveness of the negative pressure treatment. Moreover, leaks and blockages in some of the components of the NPWT apparatus may need to be detected and corrected to maintain effective treatment. For example, a leak or blockage in the tube that connects the negative pressure source to the dressing covering the wound site may disrupt the reduced pressure treatment being administered to the wound site.

Some of the currently known NPWT systems incorporate a blockage detecting functionality in order to prevent such blockages from negatively affecting the NPWT treatment. However, some of these currently known NPWT systems rely on additional components (e.g. additional pressure sensors, secondary pressure monitoring conduits, etc.) in order to provide blockage detection functionality. Moreover, in dual-lumen NPWT systems it may be difficult to discern in which of the lumens that is blocked, i.e., if it is the lumen between the negative pressure source and the wound site ("exudate lumen"), or if it is in the lumen between the electronically controllable valve and the wound site ("air lumen").

### SUMMARY

The herein disclosed technology seeks to mitigate, alleviate or eliminate one or more of the above-identified deficiencies and disadvantages in the prior art to address various problems relating to blockage detection in an NPWT system.

Various aspects and embodiments of the disclosed technology are defined below and in the accompanying independent and dependent claims.

An aspect of the disclosed technology comprises an apparatus for providing negative pressure to a wound site. The apparatus comprises a source of negative pressure configured to provide negative pressure via a first fluid flow path from an inlet of the source of negative pressure to a wound site, and an electronically controllable valve configured to release negative pressure from the wound site via a second fluid flow path. The apparatus further comprises a pressure sensor configured to measure a pressure level in the second fluid flow path, and control circuitry operatively connected to the source of negative pressure, the electronically controllable valve, and the pressure sensor. The control circuitry is configured to open the electronically controllable valve, and in response to a pressure change rate being above a pressure change rate value, output a signal indicative of a blockage in the second fluid flow path. The control circuitry is further configured to in response to the pressure change rate being below the pressure change rate value, close the electronically controllable valve and activate the source of negative pressure, and in response to the pressure level, as measured by the pressure sensor, being non-responsive to a change in negative pressure generated while the source of negative pressure is active, output a signal indicative of a blockage in the first fluid flow path.

Another aspect of the disclosed technology comprises a (computer-implemented) method for operating an apparatus for providing negative pressure to a wound site. The apparatus comprises a source of negative pressure configured to provide negative pressure via a first fluid flow path from an inlet of the source of negative pressure to a wound site, an electronically controllable valve configured to release negative pressure from the wound site via a second fluid flow path, and a pressure sensor configured to measure a pressure level in the second fluid flow path. The method comprises opening the electronically controllable valve, and in response to a pressure change rate being above a pressure change rate value, outputting a signal indicative of a blockage in the second fluid flow path. The method further comprises in response to the pressure change rate being below the pressure change rate value, closing the electronically controllable valve and activating the source of negative pressure, and in response to the pressure level, as measured by the pressure sensor, being non-responsive to a change in negative pressure generated while the source of negative pressure is active, outputting a signal indicative of a blockage in the first fluid flow path. With this aspect of the disclosed technology, similar advantages and preferred features are present as in the other aspects.

Another aspect of the disclosed technology comprises a computer program product comprising instructions which, when the program is executed by a computing device of an apparatus for providing negative pressure to a wound dressing, causes the apparatus to carry out the method according to any one of the embodiments disclosed herein. With this aspect of the disclosed technology, similar advantages and preferred features are present as in the other aspects.

Another aspect of the disclosed technology comprises a (non-transitory) computer-readable storage medium comprising instructions which, when executed by a computing device of an apparatus for providing negative pressure to a wound dressing, causes the apparatus to carry out the method according to any one of the embodiments disclosed herein. With this aspect of the disclosed technology, similar advantages and preferred features are present as in the other aspects.

The term "non-transitory," as used herein, is intended to describe a computer-readable storage medium (or "memory") excluding propagating electromagnetic signals, but are not intended to otherwise limit the type of physical computer-readable storage device that is encompassed by the phrase computer-readable medium or memory. For instance, the terms "non-transitory computer readable medium" or "tangible memory" are intended to encompass types of storage devices that do not necessarily store information permanently, including for example, random access memory (RAM). Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link. Thus, the term "non-transitory", as used herein, is a limitation of the medium itself (i.e., tangible, not a signal) as opposed to a limitation on data storage persistency (e.g., RAM vs. ROM).

Another aspect of the disclosed technology comprises a wound treatment system comprising apparatus according to any one of the embodiments disclosed herein, a wound cover for creating a sealed space defined in part by the wound site, and a tubing assembly defining the first fluid flow path and the second fluid flow path.

The disclosed aspects and preferred embodiments may be suitably combined with each other in any manner apparent to anyone of ordinary skill in the art, such that one or more features or embodiments disclosed in relation to one aspect may also be considered to be disclosed in relation to another aspect or embodiment of another aspect.

An advantage of some embodiments is that blockage conditions in NPWT systems may be detected in a reliable and robust manner.

An advantage of some embodiments is that it is possible to discern if the blockage is in an "exudate lumen" or an "air lumen" of a dual lumen NPWT system.

An advantage of some embodiments is that the energy consumption of the NPWT apparatus may be reduced over an entire treatment period.

Further embodiments are defined in the dependent claims. It should be emphasized that the term "comprises/comprising" when used in this specification is taken to specify the presence of stated features, integers, steps, or components. It does not preclude the presence or addition of one or more other features, integers, steps, components, or groups thereof.

These and other features and advantages of the disclosed technology will in the following be further clarified with reference to the embodiments described hereinafter.

### BRIEF DESCRIPTION OF THE DRAWINGS

The above aspects, features and advantages of the disclosed technology, will be more fully appreciated by reference to the following illustrative and non-limiting detailed description of example embodiments of the present disclosure, when taken in conjunction with the accompanying drawings, in which:
Fig. 1 is a schematic illustration of a wound treatment system in accordance with some embodiments.
Fig. 2 is a schematic graph of negative pressure over time for a pressure regulation cycle in accordance with some embodiments.
Fig. 3 is a schematic graph of negative pressure over time for a pressure regulation cycle in accordance with some embodiments.
Fig. 4 is a schematic graph of negative pressure over time for multiple pressure regulation cycles in accordance with some embodiments.
Fig. 5 is a schematic graph of negative pressure over time for multiple pressure regulation cycles in accordance with some embodiments.
Fig. 6 is a schematic graph of negative pressure over time for multiple pressure regulation cycles in accordance with some embodiments.
Fig. 7 is a schematic graph of negative pressure over time for multiple pressure regulation cycles in accordance with some embodiments.
Fig. 8 is a schematic flowchart representation of a method for operating an apparatus for providing negative pressure to a wound site in accordance with some embodiments.

### DETAILED DESCRIPTION

The present disclosure will now be described in detail with reference to the accompanying drawings, in which some example embodiments of the disclosed technology are shown. The disclosed technology may, however, be embodied in other forms and should not be construed as limited to the disclosed example embodiments. The disclosed example embodiments are provided to fully convey the scope of the disclosed technology to the skilled person. Like reference characters refer to like elements throughout. Those skilled in the art will appreciate that the steps, services and functions explained herein may be implemented using individual hardware circuitry, using software functioning in conjunction with hardware circuitry such as a programmed microprocessor or general-purpose computer, using one or more Application Specific Integrated Circuits (ASICs), using one or more Field Programmable Gate Arrays (FPGA) and/or using one or more Digital Signal Processors (DSPs).

It will also be appreciated that when the present disclosure is described in terms of a method, it may also be embodied in an apparatus comprising one or more processors, one or more memories coupled to the one or more processors, where computer code is loaded to implement the method. For example, the one or more memories may store one or more computer programs that cause the apparatus to perform the steps, services and functions disclosed herein when executed by the one or more processors in some embodiments.

It is also to be understood that the terminology used herein is for purpose of describing particular embodiments only, and is not intended to be limiting. It should be noted that, as used in the specification and the appended claim, the articles "a", "an", "the", and "said" are intended to mean that there are one or more of the elements unless the context clearly dictates otherwise. Thus, for example, reference to "a unit" or "the unit" may refer to more than one unit in some contexts, and the like. Furthermore, the words "comprising", "including", "containing" do not exclude other elements or steps. It should be emphasized that the term "comprises/comprising" when used in this specification is taken to specify the presence of stated features, integers, steps, or components. It does not preclude the presence or addition of one or more other features, integers, steps, components, or groups thereof. The term "and/or" is to be interpreted as meaning "both" as well and each as an alternative. Similarly, "at least one of A and B" is to be interpreted as only A, only B, or both A and B.

It will also be understood that, although the term first, second, etc. may be used herein to describe various elements or features, these elements should not be limited by these terms. These terms are only used to distinguish one element from another. For example, a first signal could be termed a second signal, and, similarly, a second signal could be termed a first signal, without departing from the scope of the embodiments. The first signal and the second signal are both signals, but they are not the same signal. The term "operatively connected" is in the context of the present disclosure to be understood as that the "operatively connected" entities or units can transmit and/or receive signals to and/or from each other.

As used herein, the term "in response to" may be construed to mean "when or "upon" or "if" depending on the context. Similarly, the phrase "if it is determined' or "when it is determined" or "in an instance of" may be construed to mean "upon determining or "in response to determining" or "upon detecting and identifying occurrence of an event" or "in response to detecting occurrence of an event" depending on the context. Accordingly, the phrase "if X equals Y" may be construed as "when X equals Y", "when it is determined that X equals Y", "in response to X being equal to Y", or "in response to detecting/determining that X equals Y" depending on the context.

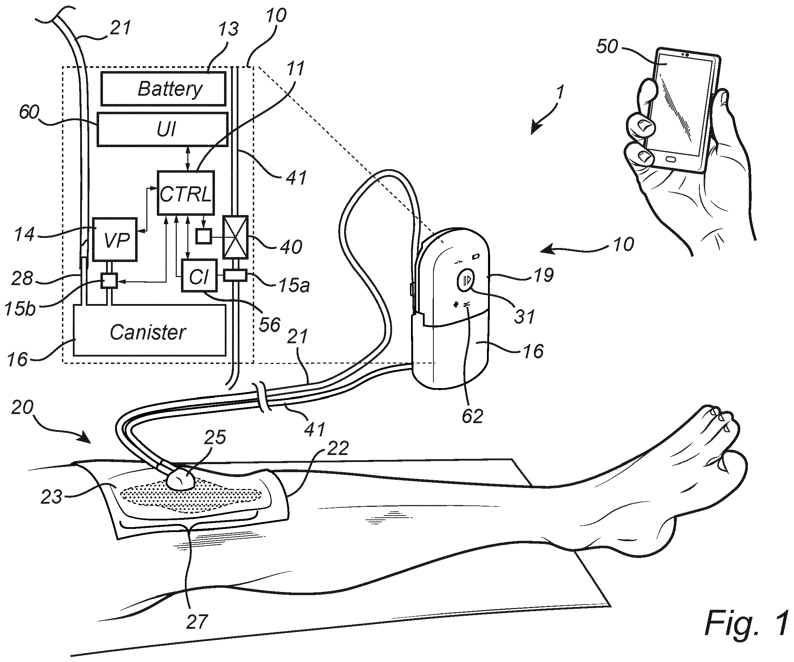

Turning now to the drawings, and to Fig. 1 in particular, there is schematically illustrated a wound treatment system 1 in accordance with some embodiments. The depicted wound treatment system 1 may be referred to as an "NPWT system" 1, "reduced pressure wound treatment system" 1, or "negative pressure wound treatment system" 1. In particular, the depicted wound treatment system 1 may be referred to as a dual lumen wound treatment system 1. The wound treatment system 1 comprises an apparatus 10 for providing negative pressure to a wound site 27 (or otherwise referred to as a tissue site 27). The wound treatment system 1 further comprises a wound cover 22 that is adapted to create a sealed space 23 defined in part by a wound surface, such as at the skin of a user/person, at or around a wound of the user/person.

Further, the apparatus 10 (may also be referred to as an NPWT device 10) is fluidly connected to the wound cover 22 using a tubing assembly with a first tubing 21 at least partly defining a first fluid flow path and a second tubing 41 at least partly defining a second fluid flow path. The tubing 21, 41 may be of any suitable flexible tubing fabricated from elastomeric or polymeric materials. The tubing 21, 41 may connect to the wound dressing 20, or more specifically to the wound cover 22 of the wound dressing 20, via a suitable connector 25. The connector 25 may be adhered or otherwise attached to the wound cover 22. In particular, the connector 25 may be attached around an opening (not shown) formed in the wound cover 22. The tubing assembly may accordingly comprise two individual tubes.

The expression "wound cover" as used herein should be interpreted broadly as any wound site member, e.g. it can be a film sealed around a periphery of a wound site 27, where a wound filler may be used to fill the wound volume prior to the application of such wound cover. Wound cover may also refer to a backing layer of a wound dressing 20 comprising an additional layer(s) such as for example an absorbent layer and/or a spacer layer.

The apparatus 10 comprises a source of negative pressure 14 configured to provide negative pressure (i.e. sub-atmospheric pressure) via a first fluid flow path from an inlet of the source of negative pressure 14 to the wound dressing 20, the wound site 27, the sealed space 23, or to the wound cover 22. The first fluid flow path ("exudate lumen") is at least partly defined by the first tubing 21. The wound treatment system 1 further comprises a second fluid flow path that fluidly connects the wound site, or the sealed space 23 created by the wound cover 22, to the ambient atmosphere or a "fluid reservoir" (not shown) via an electronically controllable valve 40. The second fluid flow path ("air lumen") is at least partly defined by the second tubing 41. Here, the fluid flow path from the inlet of the source of negative pressure 14 to the wound site 27 may be construed as a first fluid flow path or "exudate lumen".

The source of negative pressure 14 is indicated as "VP" ("Vacuum pump") in the figure. In some embodiments, the source of negative pressure 14 comprises a negative pressure pump that together with a motor is adapted for establishing a negative pressure when the source of negative pressure 14 is operating, i.e. when it is in an active state or simply "active". The source of negative pressure may comprise any type of pump and motor that are biocompatible or otherwise suitable for use in an NPWT setting and capable of maintaining or drawing adequate and therapeutic vacuum levels. Preferably, the negative pressure level to be achieved is in a range between about -20 mmHg and about -300 mmHg. In some embodiments, a negative pressure range between about -80 mmHg and about -140 mmHg is used. In some embodiments, a negative pressure range between about -115 mmHg and about -135 mmHg is used. In some embodiments, the negative pressure pump is a diaphragm pump, a peristaltic pump, piezoelectric pump or the like, in which a motor causes the moving parts to draw fluid from the wound site 27.

In the context of the present disclosure, it should be understood that the expressions "negative pressure", "sub-atmospheric pressure", "reduced pressure", or even "vacuum", as used interchangeably herein, generally refer to a pressure less than a local ambient pressure, such as the ambient pressure in a local environment external to a sealed therapeutic environment provided by a wound cover 22 or dressing 20. In many cases, the local ambient pressure may also be the atmospheric pressure at which a patient is located. Thus, the "negative pressure" may be understood as a pressure difference between the ambient pressure and the pressure under the wound cover, where ambient pressure is generally set as 0 mmHg. Unless otherwise indicated, values of pressure stated herein are gauge pressures. Similarly, references to increases in negative pressure typically refer to a decrease in absolute pressure, while decreases in negative pressure typically refer to an increase in absolute pressure.

Further, in some embodiments the apparatus 10 comprises a canister 16 fluidly connected to the negative pressure source 14. The canister 16 may be formed from e.g. moulded plastic or the like, and possibly be a detachable component of the apparatus 10. Moreover, the canister 16 may further be at least partly transparent/translucent to permit viewing into the interior of the canister 16 to assist the user in determining the remaining capacity of the canister 16. As used herein, the term "fluidly connected" should be interpreted broadly and may comprise e.g. any form of tubing, conduits, or channels providing a fluid connection/communication between two components such as between the canister 16 and the negative pressure source 14 or the canister 16 and the wound dressing 20.

In some embodiments, the canister 16 comprises an inlet port 28 for allowing connection to the tubing 21. The inlet port 28 may also be formed elsewhere at the apparatus 10, however still fluidly connected to the canister 16. The connection between the inlet port 28 and the tubing 21 is a sealed connection, thus ensuring that no leakage is formed at the inlet port 28 during normal operation of the apparatus 10. The tubing 21 is preferably detachably connected to the inlet port 28 through conventional means including a friction fit, bayonet coupling, snap fit, barbed connector, or the like. The inlet port 28 may be moulded/formed from the same material and/or at the same time as forming the canister 16. A similar sealed connection (e.g. using a flange insulation/"O-ring") is formed between the canister 16 (at the outlet port 29) and the source of negative pressure 14. The canister 16 may form a part of the first fluid flow path.

In some embodiments, the apparatus comprises a housing 19 enclosing the negative pressure source 14. The canister 16 may be detachably connected to a housing 19 comprising the negative pressure source 14, whereby e.g. a full canister may be removed and replaced with an empty (new) canister. In such embodiments it may be desirable to provide e.g. the canister 16 and the housing 19 with some form of engagement means for securing the canister 16 to the housing such that the canister 16 is not unintentionally removed from the housing 19. The engagement means may in one embodiment comprise a pair of flexible protrusions extending from the canister 16 and adapted to engage with e.g. corresponding locking grooves provided at the housing 19.

However, in some embodiments, the wound treatment system 1 is a canister-less wound treatment system (not shown), where the wound exudate is collected in an absorbent layer, or the like, of the wound dressing 20. In such embodiments, the fluid flow path from the wound dressing 20 to the negative pressure source 14 is provided with one or more suitable filters (not shown) that are adapted to allow gas flow from the wound site 27 to the source of negative pressure 14 and block the flow of liquids from the wound site 27 to the source of negative pressure 14, as readily understood by the person skilled in the art.

In some embodiments, the apparatus 10 comprises a power source, such as e.g. a battery 13 for powering the apparatus 10. The battery 13 may preferably be of the rechargeable type but may alternatively be arranged to be disposable and thus to be changed once discharged. A specifically adapted battery pack may be used in relation to some embodiments. The apparatus 10 may be of single-use type (allowing use during one single treatment time duration) or multiple-use type (allowing use during several different treatment sessions).

Furthermore, the apparatus 10 comprises a pressure sensor 15a arranged in fluid connection with the second fluid flow path. In particular, the apparatus 10 comprises at least one pressure sensor 15a configured to measure a pressure level in the second fluid flow path. In some embodiments, the apparatus 10 comprises a single pressure sensor 15a configured to measure/monitor a pressure level in the second fluid flow path. In the depicted embodiment a pressure sensor 15a is arranged within the housing downstream of the electronically controllable valve 40 (i.e., towards the wound site from the valve 40). However, as readily understood by the skilled person in the art, the one or more pressure sensors 15a may be arranged at any other suitable location to sense or detect a pressure within the second fluid flow path ("air lumen"). The apparatus 10 may comprise additional pressure sensors. In some embodiments, the apparatus 10 comprises one or more (secondary) pressure sensors 15b arranged in fluid connection with the inlet of the source of negative pressure 14, the canister 16, and/or the sealed space 23. In the depicted embodiment of Fig. 1, the apparatus 10 comprises a (secondary) pressure sensor 15b arranged in a fluid flow path between the canister 16 and the source of negative pressure 14 (i.e., in fluid connection with the first fluid flow path). However, as readily understood by a person skilled in the art, the (secondary) pressure sensor 15b may be located at any other suitable location to sense or detect a pressure within the fluid flow path between the source of negative pressure 14 and the wound site 27. For example, the (secondary) pressure sensor 15b may be arranged within the canister or within the wound dressing 20.

The apparatus 10 further comprises control circuitry "CTRL" 11 (may also be referred to as "a control unit", "a controller", or the like) operatively connected to the battery 13, the source of negative pressure 14, the electronically controllable valve 40, and the pressure sensor 15a. The control circuitry 11 is configured to control operation of the source of negative pressure 14 and the electronically controllable valve 40. The control circuitry 11 may comprise a microprocessor, microcontroller, programmable digital signal processor or another programmable device. The control circuitry 11 may also, or instead, include an application specific integrated circuit (ASIC), a programmable gate array or programmable array logic, a programmable logic device, or a digital signal processor. Where the control circuitry 11 includes a programmable device such as the microprocessor, microcontroller or programmable digital signal processor mentioned above, the control circuitry 11 may further include computer executable code that controls operation of the programmable device. Thus, in some embodiments, the control circuitry 11 comprises one or more processors and a memory, where the memory contains instructions executable by the processor whereby the apparatus 10 is operative to execute any one of the method steps or functions disclosed herein. The term "electronically controllable" should in present context be understood as that one can control the unit or component that is "electronically controllable" with electric signals (control signals), and in particular with electric signals that are output from the control circuitry 11.

During use of the apparatus 10, the wound cover 22 is arranged at a wound site 27 of the user/patient, forming the sealed space 23. The tubing 21 is provided to fluidly connect the wound cover 22 to the inlet port 28 of the apparatus 10. The apparatus 10 is then activated, e.g. by a user pressing a start/pause button 31 of a user-interface 60 of the apparatus 10. The source of negative pressure 14 is thereby activated. When activated, and the valve 40 is closed, the source of negative pressure 14 will start to evacuate air through the canister 16, the inlet port 28, the tubing 21 and the sealed space 23 formed by the wound cover 22. Accordingly, a negative pressure will be created within the sealed space 23. In some embodiments, the control circuitry 11 is configured to control the source of negative pressure 14 so to establish and maintain a negative pressure level (as measured by the pressure sensor(s) 15a, 15b) of negative pressure in a negative pressure range. The negative pressure range may for example be -20 mmHg to -300 mmHg, -80 to -180 mmHg, -100 to -150 mmHg, -110 to -140 mmHg, or -115 to -135 mmHg. The negative pressure range may for example be selected in view of the type of wound and/or the patient.

In case a liquid has been formed at the wound site 27, this liquid from the wound site 27 may at least partly be "drawn" from the wound site, through the tubing 21, the inlet port 28 and into the canister 16. The amount of liquid (possibly defined as exudate) that is drawn from the wound and collected in the canister 16 will depend on the type of wound that is being treated, the duration of the treatment, as well as the type of wound dressing 20 used. For example, in case an absorbent dressing is used, the liquid may be absorbed and collected both in the canister 16 and the wound dressing 20, whereas if a dressing with no absorption capacity or little absorption capacity is used, most or all of the liquid from the wound site 27 may be collected in the canister 16. A suitable filter member (not shown) is generally arranged between at the outlet port 29 of the canister 16 to ensure that no liquid is allowed to pass to the source of negative pressure 14 from the canister 16.

The control circuitry 11 is further configured to control an operation of the electronically controllable valve 40 so to release negative pressure from the wound site via the second fluid flow path (or "air lumen") 41. In other words, the control circuitry 11 is configured to open the electronically controllable valve 40 so to introduce fluid (e.g. air) to the wound site 27 when there is a sub-atmospheric pressure under the wound cover 22. This operation may also be referred to as "flushing". Since the wound treatment system 1 depicted in Fig. 1 does not have any "controlled leakage flow", there is a risk that the apparatus 10 would not be able to draw any, or at least a sub-optimal amount of "exudate" from the wound site 27 to the canister 16 once negative pressure has been established under the wound cover 22 due to lack of airflow through the system 1. Therefore, in order to be able to draw desired amounts of wound exudate from the wound site 27, a fluid, such as e.g. air from the ambient atmosphere, is controllably introduced at defined time intervals and/or in response to pressure measurements by opening the electronically controllable valve 40. In general, once enough fluid has been introduced (e.g. in response to a negative pressure within the first and/or second fluid flow path reaching a lower negative pressure threshold), the control circuitry 11 is configured to close the electronically controllable valve 40 and to activate the source of negative pressure 14. However, in some embodiments, the negative pressure source 14 may be active for at least some of the duration that the electronically controllable valve 40 is open.

The ability to controllably "flush" the system 1 by injecting air via an electronically controllable valve 40 may provide the advantage of longer pressure regulation cycles (may also be referred to as NPWT cycle), which reduces the on-time for the source of negative pressure 14 and therefore reduces the energy consumption of the apparatus 10.

The apparatus 10 may further comprise an air filter (not shown) arranged in the second fluid flow path in order to limit the airflow when the electronically controllable valve 40 is open. In some embodiments, the air filter comprises a hydrophobic and porous material, where the size of the pores is configured to allow for a flow in the range of 50-120 ml/s, such as 60-100 ml/s when the valve 40 is opened after negative pressure has been established under the wound cover 20. The pore size of the filter is preferably measured in a non-compressed state. In some embodiments, the air filter comprises polyethylene or sintered polyethylene. The air filter may also serve to reduce the risk of contaminants entering the wound site 27 when the valve 40 is opened (i.e., during "flushing"). The air filter may be arranged in the tube 41 that at least partly defines the second fluid flow path. The air filter may be arranged upstream of the valve 40 (i.e., between the valve 40 and the inlet of ambient air, or downstream of the valve 40 such as between the valve and the pressure sensor 15a.

In some embodiments, during use, the control circuitry 11 is configured to regulate the negative pressure provided to the wound site over a plurality of pressure regulation cycles. In general, a pressure regulation cycle may be understood as a time period comprising at least a "pressure regulation period" and a "flushing period". During the "pressure regulation period" the control circuitry 11 is configured to operate the negative pressure source 14 so to maintain a level of negative pressure in a suitable negative pressure range as exemplified above, i.e., the apparatus 10 is operated in a "pressure regulation mode". In other words, during the "pressure regulation period" the electronically controllable valve 40 is closed and the source of negative pressure 14 is operated to maintain a desired level of negative pressure at the wound site 27.

During the "flushing period" the control circuitry 11 is configured to open the electronically controllable valve 40 so to introduce fluid (e.g., air from the ambient atmosphere) to the wound site 27, close the valve 40, and activate the source of negative pressure 14 to draw wound exudate from the wound site and re-establish the negative pressure level, i.e., the apparatus 10 is operated in a "flushing mode". Accordingly, the flushing period causes a temporary decrease in negative pressure at the wound site 27 (i.e., a temporary increase in absolute pressure).

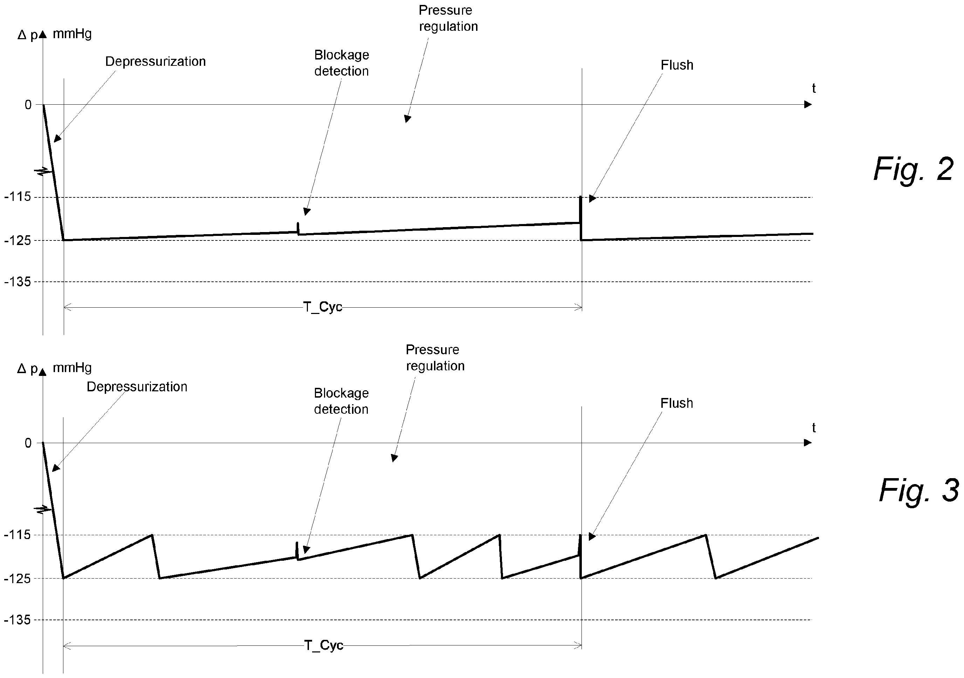

The durations of the pressure regulation periods and flushing periods, and accordingly the duration of a pressure regulation cycle, may be fixed (i.e., time-controlled) or dynamically controlled based pressure measurements. The duration of a pressure regulation cycle may also be dynamically controlled based on a total treatment time such that the pressure regulation cycles are shorter during an initial part of the treatment as compared to a later part. However, in some embodiments, the durations of the pressure regulation periods and flushing periods, and accordingly the duration of a pressure regulation cycle, may be controlled based on pressure measurements in combination with time-control. For example, during the flushing period the control circuitry 11 may be configured to open the valve until a pressure level P (as measured by the sensor(s) 15a, 15b) is reached or for 2 seconds, whichever occurs first. Thus, the time-control aspect may define a longest duration for each period, while detection of certain pressure levels may shorten the duration. Further examples and details related to the pressure regulation cycle are provided in reference to Figs. 2-7 below.

Moving on, the apparatus 10 is provided with a blockage-detection functionality in order to alert a user (i.e., a patient or a caregiver) of the apparatus 10 of the existence of a blockage condition in the wound treatment system 1. A blockage condition may be caused by an external impact such as a patient lying on a tube 21, 41 or a patient bending the tubing 41, 42. A blockage condition may also be caused by substances which are contained in the fluids sucked away from the wound (such as proteins) that may clog the tubing 21, 41. Even though many of the existing blockage-functionalities for dual lumen NPWT systems are capable of detecting blockage-conditions, they generally struggle with discerning if the blockage is in the air lumen or in the exudate lumen, or require additional components that add to the cost and weight of the system 1.

Accordingly, in order to detect a blockage condition in the wound treatment system 1, the control circuitry 11 may be configured to execute a blockage-detection function or operate the apparatus 10 in a blockage-detection mode. In some embodiments, the blockage-detection function may be understood as a "mini-flush" operation while monitoring the pressure level measured by the pressure sensor 15a associated with the "air lumen".

In more detail, the control circuitry 11 may be configured to open the electronically controllable valve 40 at a time after negative pressure has been established at the wound site (i.e., under the wound cover), and in response to a pressure change rate being above a pressure change rate value, output a signal indicative of a blockage in the second fluid flow path. Furthermore, the control circuitry 11 is configured to in response to the pressure change rate being below the pressure change rate value, close the electronically controllable valve 40 and activate the source of negative pressure 14, and in response to the pressure level, as measured by the pressure sensor, being non-responsive to a change in negative pressure generated while the source of negative pressure 14 is active, output a signal indicative of a blockage in the first fluid flow path. Hereby, the apparatus 10 may not only detect potential blockages in the system 1, it may also discern between a blockage in the exudate lumen 21 and a blockage in the air lumen 41.

Moreover, the term "pressure change rate value" may be understood as "a pressure change rate threshold" or "a set value of pressure change rate". The pressure change rate value/threshold may for example be a value in the range of 3 mmHg/s to 10 mmHg/s, or more preferably in the range of 4 mmHg/s to 8 mmHg/s, such as e.g., 4 mmHg/s or 5 mmHg/s.

However, in some embodiments the pressure change rate value/threshold may be defined based on a nominal/normal pressure change rate value. The nominal/normal pressure change rate value may be defined in design-time. In more detail, the nominal/normal pressure change rate value may be understood as the pressure change rate value that occurs when the valve 40 is opened and the system is free of any blockages. For example, a wound treatment system having a system volume of 400 ml and an air filter limiting the airflow to 70 ml/s through the second tubing 41 during flushing may have a nominal/normal pressure change rate of 2 mmHg/s. Thus, in some embodiments the pressure change rate value/threshold is at least twice the nominal/normal pressure change rate value, where the nominal/normal pressure change rate value is a pressure change rate measured by the pressure sensor 15a during flushing in a blockage-free system 1.

The term "in response to a pressure change rate being above a pressure change rate value" may be understood as "in response to the pressure level over time, as measured by the pressure sensor 15a over time, exceeding a pressure change rate value" or "in response to detecting a pressure change rate exceeding a pressure change rate value, where the pressure change rate is derived based on an output from the pressure sensor 15a over time". Similarly, the term "in response to the pressure level, as measured by the pressure sensor, being non-responsive to a change in negative pressure" may be understood as "in response to the pressure level, as derived based on an output from the pressure sensor 15a over time, being non-responsive to a change in negative pressure" or "in response to the pressure sensor 15a not detecting/outputting a change in negative pressure, while the source of negative pressure 14 is active".

The term "while the source of negative pressure is active" may be understood as, "while detecting that the source of negative pressure is active", "while detecting a current draw from the source of negative pressure", or similar as readily understood by the skilled person in the art. Accordingly, the apparatus 10 may comprise one or more current sensors (not shown) operable to detect a current draw of the source of negative pressure. The control circuitry 11 may accordingly be operably connected to the current sensor, and configured to determine a state of the source of negative pressure (e.g., active or inactive). Moreover, the control circuitry 11 may be configured to detect a magnitude of current being drawn by the source of negative pressure 14 based on the output from the current sensor.

During the first part of the blockage-detection mode, i.e., while the valve 40 is open, if the pressure change rate is high (i.e., above some set value), it is interpreted as an indicator that the volume of the system from the perspective of the pressure sensor 15a is low. A blockage in the second fluid flow path would decrease the volume of the system to a greater extent than a blockage in the first fluid flow path from the perspective of the pressure sensor 15a that is configured to measure a pressure level in the second fluid flow path. Here, the volume of the system 1 ("system volume") comprises the combined volume of fluid flow paths defined by the tubing assembly, the canister 16, and the volume under the wound cover 22.

Analogously, if the pressure change rate during this first part of the blockage-detection mode is within tolerable limits, it is concluded that there is no blockage in the second fluid flow path. However, it is still uncertain if there is any blockage in the first fluid flow path. Therefore, during the second part of the blockage-detection mode, i.e., while valve 40 is closed and pump 14 is active, a check is performed to see if the pressure sensor 15a detects any change in negative pressure. Here, if the pressure level, as measured by the pressure sensor 15a is concluded to be non-responsive (i.e., no change in negative pressure is detected or a small change in negative pressure is detected below some set value is detected), then it is concluded that there is a blockage in the first fluid flow path.

Moreover, in some embodiments, the blockage-detection function may be executed in accordance with the following sequence. In more detail, the control circuitry 11 may be configured to in response to a pressure level, as measured by the pressure sensor 15a, being non-responsive to a change in negative pressure generated while the source of negative pressure 14 is active, open the electronically controllable valve 40. Accordingly, if the pressure sensor's output 15a is non-responsive while the source of negative pressure 14 active, the valve 40 may be opened. Then, the control circuitry 11 may be further configured to, in response to the pressure change rate being above a pressure change rate value, output a signal indicative of a blockage in the second fluid flow path. Further, the control circuitry 11 may be configured to in response to the pressure change rate being below a pressure change rate value, output a signal indicative of a blockage in the first fluid flow path. Thereby another blockage-detection function capable of discerning blockages in the two separate fluid flow paths is provided. Moreover, the control circuitry 11 may be configured to close the valve, either after the pressure change rate measurements or after the blockage indication has been output.

In some embodiments, the apparatus 10 comprises a user-interface "Ul" 60 comprising a Light Emitting Diode (LED) 62. The user-interface 60 and the LED 62 may be operatively connected to the control circuitry 11. The LED 62 may for example be arranged on the housing 19 of the apparatus 10. The LED 62 is arranged to indicate a presence of a blockage to a user of the apparatus 10. Thus, the LED may be understood as a blockage-indicator for a user of the apparatus. Accordingly, the control circuitry 11 may be further configured to activate the LED 62 in response to the signal, indicative of a blockage in the second fluid flow path, being output, and to activate the LED 62 in response to the signal, indicative of the blockage in the first fluid flow path, being output. In other words, if a blockage is detected in either of the fluid flow paths, the LED 62 is activated so to alert a user of the apparatus 10.

Moreover, in some embodiments, the apparatus 10 comprises a user-interface 60 comprising a first LED arranged to indicate a presence of a blockage being present in the first fluid flow path to a user of the apparatus 10 and a second LED arranged to indicate a presence of a blockage being present in the second fluid flow path to a user of the apparatus 10. Accordingly, the control circuitry 11 may be configured to activate the second LED in response to the signal, indicative of a blockage in the second fluid flow path, being output, activate the first LED in response to the signal, indicative of the blockage in the first fluid flow path, being output. Using two different LEDs to discern between blockage in the "air lumen" or in the "exudate lumen" may aid the user in troubleshooting during a blockage condition.

Further, in some embodiments, the apparatus 10 further comprises a communication interface "Cl" 56 having at least one antenna and at least one transceiver operatively connected to the at least one antenna. Moreover, the transceiver may be operatively connected to the control circuitry 11, and the control circuitry may be configured to transmit a first signal to an external device 50 in response to the signal, indicative of the blockage in the first fluid flow path, being output, and transmit a second signal to the external device 50 in response to the signal, indicative of a blockage in the second fluid flow path, being output.

The communication interface 56 may comprise suitable components (e.g. transceivers, antennas, filters, power amplifiers, etc.) with suitable communication protocols (e.g., Wi-Fi, Bluetooth, Bluetooth Low Energy (BLE), Zigbee, or the like) in order to establish a connection with an external device 50 and to transmit and receive wireless signals to and from the external device 50. Accordingly, the communication interface 56 is configured to transmit and receive wireless signals to and from the external handheld device 50. In more detail, the communication interface 56 comprises one or more transceivers and one or more antennas connected to the one or more transceivers. The one or more transceivers are accordingly configured to transmit signals to an external device 50 via the one or more antennas and to receive signals from an external device 50 via the one or more antennas. For example, the communications interface 56 may include a Wi-Fi transceiver for communicating via a wireless communications network or cellular or mobile phone communications transceivers. In another example, the communication interface 56 may include a short-range wireless transmitter (e.g., a Bluetooth wireless transmitter, etc.) for communicating via a short-range wireless communication transceiver.

The external device may be an external handheld device 50, such as a mobile phone (e.g. a smart phone) operatively connected to the apparatus 10. Moreover, the handheld device 50 preferably comprises corresponding hardware and software capabilities to establish a wireless communication link with the apparatus 10 and to transmit and receive signals to and from the apparatus 10. Moreover, the external device may comprise a suitable software application configured to output a Graphical User Interface (GUI) comprising a graphical representation comprising an indication of the blockage in the wound treatment system 1 via a display apparatus of the external device 50. In more detail, depending on which signal (first or second) was received by the external device 50, the graphical representation may indicate whether the blockage is in the first fluid flow path or the second fluid flow path. Similar as before, by transmitting two different signals to the external device to discern between a blockage in the "air lumen" or in the "exudate lumen" troubleshooting may be facilitated for the user during a blockage condition.

Fig. 2 and Fig. 3 are schematic graphs of negative pressure over time, Δp(t), for a pressure regulation cycle of an apparatus 10 in accordance with some embodiments. In more detail, Fig. 2 depicts a "low leakage scenario" meaning that the wound treatment system 1 has little-to-no leakage. However, Fig. 3 depicts an "elevated leakage scenario" meaning that the wound treatment system has some unwanted leakage. The unwanted leakage may for example be due to improperly applied wound cover 22 and/or leaky connections between the tubing and other components. This is indicated by the different pressure curves in Figs. 2 and 3. More specifically, in contrast to the curve in Fig. 2, the curve in Fig. 3 indicates multiple activations of the source of negative pressure during the pressure regulation period of the pressure regulation cycle.

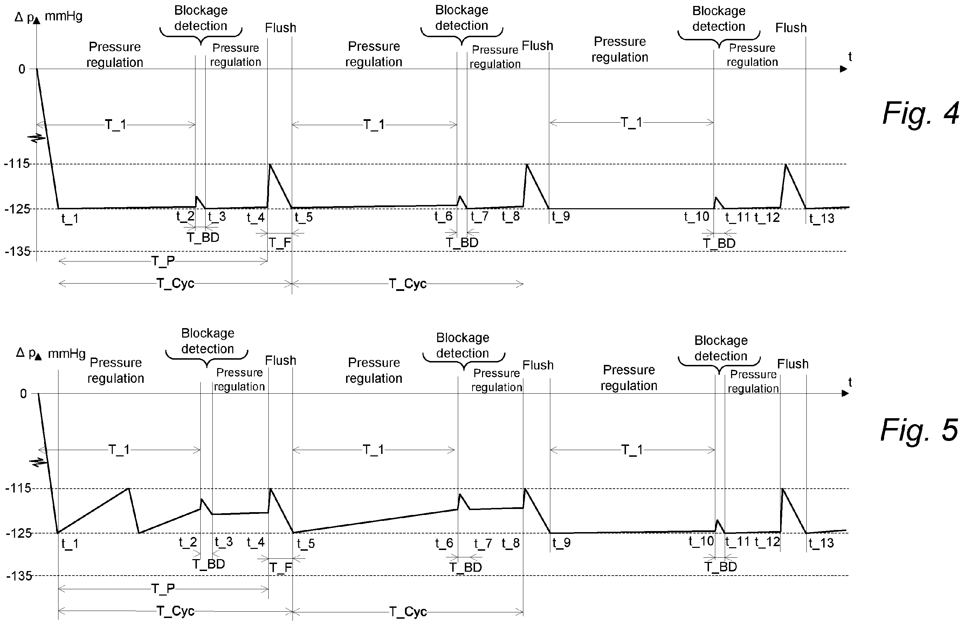

Figs. 4-7 are schematic graphs of negative pressure over time for multiple pressure regulation cycles of an apparatus 10 in accordance with some embodiments. However, in contrast to Figs. 2 and 3, the time-scale and the representations of the relative duration of each operational mode as illustrated in Figs. 4-7 is more skewed and less to-scale, whereas Figs. 2 and 3 provide a more accurate representation of the relative duration of each operational mode. Here, Figs. 4-7 are intended to provide a clearer illustration of the pressure-curve, Δp(t), for each operational mode of the apparatus 10.

Moreover, similar to Figs. 2 and 3, Fig. 4 depicts a "low leakage scenario" meaning that the wound treatment system 1 has little-to-no leakage while Fig. 5 depicts an "elevated leakage scenario" meaning that the wound treatment system has some unwanted leakage.

Further, in some embodiments, the control circuitry 11 is configured to operate the apparatus 10 in a pressure regulation mode, a flushing mode, and a blockage-detection mode. Moreover, the control circuitry 11 may be configured to operate the apparatus 10 in a depressurization mode (see e.g., Figs. 2 and 3). The depressurization mode is generally applied during an initial activation of the apparatus 10 when the pressure level at the wound site 27 is to be reduced from ambient pressure to a suitable pressure level (e.g., -125 mmHg).

The above-described blockage detection function may accordingly be executed while the apparatus 10 is operated in the blockage-detection mode. Thus, in some embodiments, the control circuitry 11 is configured to, in the blockage-detection mode, open the electronically controllable valve 40 at a time after negative pressure has been established at the wound site. This is indicated in Figs. 2-7 by the initial decrease in negative pressure (or increase in absolute pressure) during blockage detection. Then, in response to the pressure change rate being above the pressure change rate value, output the signal indicative of the blockage being present in the second fluid flow path.

Further, the control circuitry 11 is configured to, in the blockage detection mode, in response to the pressure change rate being below the pressure change rate value, close the electronically controllable valve 40 and activate the source of negative pressure 14. This is indicated in Figs. 2-7 by the latter increase in negative pressure (or increase in absolute pressure) during blockage detection. Then, in response to the pressure level, as measured by the pressure sensor 15a, being non-responsive to the change in negative pressure generated while the source of negative pressure 14 is active, output the signal indicative of the blockage being present in the first fluid flow path. Since the negative pressure increases (or absolute pressure decreases) in the depicted charts, it can be concluded that the pressure sensor 15a is responsive to the change in negative pressure generated while the source of negative pressure 14 is active (i.e., no blockage condition was detected).

Furthermore, in some embodiments, the pressure regulation mode, the flushing mode, and the blockage-detection mode are temporally separated operational modes of the apparatus 10, as for example indicated in Figs. 2-7. In other words, in some embodiments, the apparatus 10 can only be set to operate in one single operational mode at a time, meaning that there is no temporal overlap between the operational modes or that two operational modes are active concurrently.

As mentioned in the foregoing, the blockage-detection mode may be construed as a "mini-flush" mode. However, the blockage-detection mode is used for detecting blockages in the fluid flow paths rather than introducing air into the system for transporting wound exudate. Thus, in some embodiments, the blockage-detection mode operates within a first pressure range (e.g., 3 mmHg, 4 mmHg, 5 mmHg, etc.) while the flushing mode operates within a second pressure range (e.g., 10 mmHg, 15 mmHg, 20 mmHg, etc.) that is larger than the first pressure range. In other words, in some embodiments, the control circuitry 11 is configured to control the source of negative pressure 14 and the electronically controllable valve 40 so to allow the pressure level (as measured by one or more pressure sensors 15a, 15b of the apparatus 10) to vary within the first pressure range when in the blockage-detection mode, and within the second pressure range when in the flushing mode. In some embodiments, the first pressure range and second pressure range may be maximum allowable pressure ranges for each respective operational mode.

In more detail, the control circuitry 11 may be configured to, in the blockage detection mode, reduce the negative pressure by 5 mmHg (e.g., from -125 mmHg to -120 mmHg) and then increase the negative pressure by 5 mmHg (e.g., from -120 mmHg to -125 mmHg), rendering an operational pressure range of 5 mmHg for the blockage-detection mode. Similarly, the control circuitry 11 may be configured to, in the flushing mode, reduce the negative pressure by 15 mmHg (e.g., from -125 mmHg to -110 mmHg) and then increase the negative pressure by 15 mmHg (e.g., from -110 mmHg to -125 mmHg), rendering an operational pressure range of 15 mmHg for the flushing mode.

In some embodiments, a duration of the blockage-detection mode is first duration of time (e.g., 0,5 seconds, 1 second, 1,5 seconds, etc.) while a duration of the flushing mode is a second duration of time (e.g., 2 seconds, 3 seconds, 4 seconds, etc.) that is longer than the first duration of time. In other words, the control circuitry 11 may be configured to operate the apparatus 10 in the blockage-detection mode for the first duration of time and to operate the apparatus 10 in the flushing mode for the second duration of time. In some embodiments, the first duration of time and the second duration of time may be maximum allowable durations of time for each respective operational mode (i.e., for the blockage-detection mode and the flushing mode, respectively).

Moreover, the duration of the blockage-detection period (T_BD) may be time-controlled. In more detail, the electronically controllable valve 40 will be opened for X1 seconds (e.g., 0,3s) and then closed, and the source of negative pressure will be kept active or activated for X2 (e.g., 1,0s) seconds after the valve 40 has been closed, so that the total duration of the blockage-detection period is X1+X2 seconds (i.e., T_BD = X1+X2). Alternatively, the duration of the blockage-detection period (T_BD) may be pressure-controlled. In more detail, the electronically controllable valve 40 will be opened until a negative pressure level P1 (e.g., -122 mmHg) is reached (i.e., until measured by the pressure sensor 15a) and then closed, and the source of negative pressure will be kept active or activated until a negative pressure level P2 (e.g., -125 mmHg) is reached after the valve 40 has been closed.

However, in some embodiments, the duration of blockage-detection period may be both time-controlled and pressure-controlled. Thus, in some embodiments, the electronically controllable valve 40 will be opened for X3 seconds (e.g., 1s), unless a negative pressure value P3 (e.g., -120 mmHg) is reached first, and then closed. Similarly, the source of negative pressure will be kept active or activated for Y seconds (e.g., 2s) after the valve 40 has been closed, unless a negative pressure valve P4 (e.g., -125 mmHg) is reached first. Thereby the maximum total duration of the blockage-detection period is X+Y seconds and the negative pressure level as measured the pressure sensor 15a is only allowed to vary between P3 and P4.

Analogously, the electronically controllable valve 40 may be opened until a negative pressure value P3 is reached, unless the valve 40 has been open for X3 seconds, and then closed, and the source of negative pressure 14 may be be kept active or activated until a negative pressure valve P4 is reached after the valve 40 has been closed, unless the source of negative pressure 14 has been active for Y seconds. Thereby the maximum total duration of the blockage-detection period is X+Y seconds and the negative pressure level as measured the pressure sensor 15a is only allowed to vary between P3 and P4.

Accordingly, the control circuitry 11 may be configured to open the electronically controllable valve 40 for a first duration of time or until the pressure level, as measured by the pressure sensor 15a, reaches a first pressure value. Similarly, the control circuitry 11 may be configured to in response to the pressure change rate being below the pressure change rate value, close the electronically controllable valve 40 and activate the source of negative pressure 14 for a second duration of time or until the pressure level, as measured by the pressure sensor 15a, reaches a second pressure value.

Further, in some embodiments, the duration of the blockage-detection mode (i.e., the time the blockage-detection mode is allowed to be active) is less than the duration of the flushing mode (i.e., the time the flushing mode is allowed to be active). Thereby the energy consumption of the apparatus 10 for executing the blockage-detection function is less than for executing the flushing function. Accordingly, by introducing a more energy efficient blockage detection mode, the need for flushing the system 1 for blockage detection is reduced resulting in a reduced energy consumption of the apparatus 10 over a whole treatment period.

Moreover, in some embodiments, the control circuitry 11 is configured to execute the blockage-detection function or to set the apparatus 10 in the blockage detection mode at a set time (T_1) after the latest or most recent flushing function. In other words, the control circuitry 11 is configured to execute the blockage-detection function or to set the apparatus 10 in the blockage detection mode at a set time (T_1) after the end of the latest or most recent flushing function. Stated differently, the control circuitry 11 is configured to execute the blockage-detection function or to set the apparatus 10 in the blockage detection mode at a set time after the apparatus 10 completed the most recent flush cycle. The timing of the blockage detection function is indicated as T_1 in Figs. 2-7. Thus, once a flush cycle is completed, a counter may be started, and in response to that counter reaching a set value (T_1), the apparatus 10 is set into the blockage-detection mode (in order to execute the blockage detection function).

As readily understood by the skilled reader, to "execute the blockage-detection function" or to "set the apparatus into the blockage-detection mode" comprises at least opening the electronically controllable valve 40 at a time after negative pressure has been established (e.g., established at a set level) at the wound site, and, in response to the pressure change rate being above the pressure change rate value, output the signal indicative of the blockage being present in the second fluid flow path. Then, in response to the pressure change rate being below the pressure change rate value, close the electronically controllable valve 40 and activate the source of negative pressure 14. Then, in response to the pressure level, as measured by the pressure sensor 15a, being non-responsive to the change in negative pressure generated while the source of negative pressure 14 is active, output the signal indicative of the blockage being present in the first fluid flow path.

Furthermore, as indicated in Figs. 4-5, the duration of a complete pressure regulation cycle (T_Cyc) may be fixed. Thus, the blockage-detection function may be executed for each pressure regulation cycle or NPWT cycle. In other words, the control circuitry 11 may be configured to execute the blockage-detection function for each pressure regulation cycle.

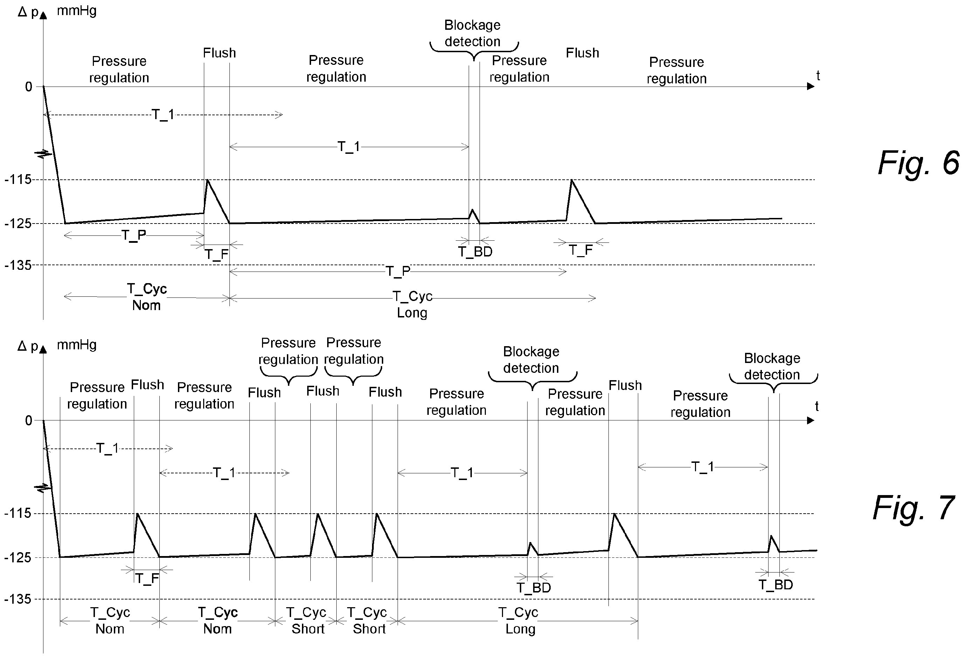

However, as indicated in Figs. 6-7, in some embodiments, the duration of a complete pressure regulation cycle (T_Cyc) may be variable or dynamically set. In other words, the flushing function may be executed at variable intervals, as opposed to fixed intervals as depicted in Figs. 4-5. This may for example be advantageous in situations where there is excess wound exudate being emitted from the wound, and therefore has to be transported from the wound site 27 to the canister 16 in a more frequent manner. Another advantage is when there is a low amount of wound exudate being emitted from the wound, then one can reduce the energy consumption of the apparatus 10, since the flushing function need not be activated frequently. In such scenarios, where the duration of a complete pressure regulation cycle (T_Cyc) is variable, and when the flushing function is executed at a set time (T_1) after the latest or most recent flushing function, the blockage-detection function is not necessarily executed for each pressure regulation cycle. However, in situations when there is a long time between flushing periods, blockage conditions may still be caught reasonably quick due to the blockage detection mode.

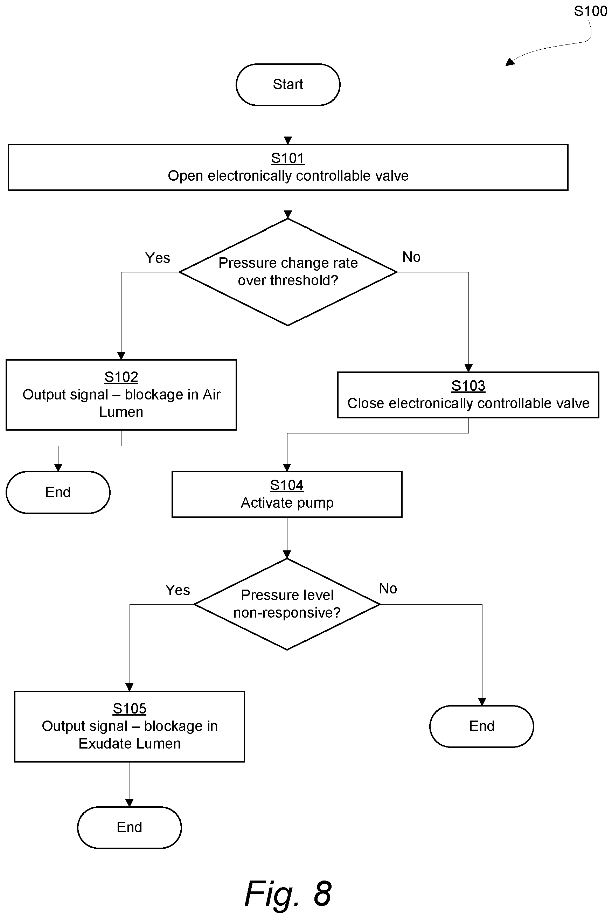

Fig. 8 is a schematic flowchart representation of a computer-implemented method S100 for operating an apparatus for providing negative pressure to a wound site in accordance with some embodiments. The apparatus may be an apparatus according to any one of the embodiments disclosed herein. However, in general, the apparatus comprises a source of negative pressure configured to provide negative pressure via a first fluid flow path from an inlet of the source of negative pressure to a wound site, an electronically controllable valve configured to release negative pressure from the wound site via a second fluid flow path, and a pressure sensor configured to measure a pressure level in the second fluid flow path. The method S100 is preferably a computer-implemented method S100, performed by a processing system of the apparatus. The processing system may for example comprise one or more processors and one or more memories coupled to the one or more processors, wherein the one or more memories store one or more programs that perform the steps, services and functions of any one of the embodiments disclosed herein when executed by the one or more processors.

The method S100 comprises opening S101 the electronically controllable valve at a time after negative pressure has been established at the wound site. Then, in response to a pressure change rate being above a pressure change rate value, outputting S102 a signal indicative of a blockage in the second fluid flow path. Further, in response to the pressure change rate being below the pressure change rate value, the method S100 comprises closing S103 the electronically controllable valve and activating the source of negative pressure, and in response to the pressure level, as measured by the pressure sensor, being non-responsive to a change in negative pressure generated while the source of negative pressure is active, outputting S105 a signal indicative of a blockage in the first fluid flow path.

Further, in some embodiments, the apparatus is configured to operate in a pressure regulation mode, a flushing mode, and a blockage-detection mode. The method S100 may further comprise during the blockage-detection mode opening S101 the electronically controllable valve, and in response to the pressure change rate being above the pressure change rate value, outputting S102 the signal indicative of the blockage being present in the second fluid flow path. Further, the method S100 may comprise, during the blockage detection mode, and in response to the pressure change rate being below the pressure change rate value, closing S103 the electronically controllable valve and activating S105 the source of negative pressure. Further, the method S100 may comprise, during the blockage detection mode, and in response to the pressure level, as measured by the pressure sensor, being non-responsive to the change in negative pressure generated while the source of negative pressure is active, outputting S105 the signal indicative of the blockage being present in the first fluid flow path. In some embodiments, the source of negative pressure may be activated S104 prior to the closing S103 of the valve. For example, the source of negative pressure may be kept active for the duration of the blockage detection mode. Further, in some embodiments, the method S100 comprises executing the blockage-detection function or to setting the apparatus 10 in the blockage detection mode at a set time (T_1) after the latest or most recent flushing function.

However, in some embodiments, the blockage detection function as described herein may be executed in response to another, different, blockage detection functionality indicating a blockage condition, whereupon the blockage detection function as described herein may be utilized to discern if the blockage is in the exudate lumen or in the air lumen. Thus, the blockage detection function as described herein may be used as a post-hoc analysis to discern if the reported blockage condition is due to a blockage in the exudate lumen or in the air lumen. Other blockage detection functionalities may for example include blockage detection functionalities that are triggered based on pump motor activations and/or pressure readings. Thereby improved robustness and granularity in the overall blockage detection function of the apparatus may be achievable.

For example, in some embodiments, the method S100 may comprise in response to a pressure level, as measured by the pressure sensor, being non-responsive to a change in negative pressure generated while the source of negative pressure is active, open the electronically controllable valve. Accordingly, if the pressure sensor's output is non-responsive while the source of negative pressure active, the valve may be opened as this may be indicative of that a blockage condition is present. Then, a sequence may be run in order to discern if the indicate blockage is due to a blockage in the exudate lumen or in the air lumen. Accordingly, the method S100 may further comprise in response to the pressure change rate being above a pressure change rate value, outputting a signal indicative of a blockage in the second fluid flow path. Further, the method S100 may comprise in response to the pressure change rate being below a pressure change rate value, outputting a signal indicative of a blockage in the first fluid flow path. Moreover, the method S100 may comprise closing the valve, either after the pressure change rate measurements or after the blockage indication being output.

Further, in some embodiments, the apparatus further comprises a user-interface comprising a Light Emitting Diode (LED) arranged to indicate a presence of a blockage to a user of the apparatus. Thus, outputting S102 the signal indicative of the blockage being present in the second fluid flow path may comprise activating the LED. Similarly, outputting S105 the signal indicative of the blockage being present in the first fluid flow path may comprise activating the LED.

However, in some embodiments, the apparatus further comprises a user-interface comprising a first LED arranged to indicate a presence of a blockage being present in the first fluid flow path to a user of the apparatus and a second LED arranged to indicate a presence of a blockage being present in the second fluid flow path to a user of the apparatus. Thus, outputting S102 the signal indicative of the blockage being present in the second fluid flow path may comprise activating the second LED, and outputting S105 the signal indicative of the blockage being present in the first fluid flow path may comprise activating the first LED.

In some embodiments, the apparatus further comprises a communication interface having at least one antenna and at least one transceiver operatively connected to the at least one antenna. Accordingly, the method S100 may further comprise transmitting a first signal to an external device in response to the signal, indicative of the blockage in the first fluid flow path, being output S105. The method S100 may further comprise transmitting a second signal to the external device in response to the signal, indicative of a blockage in the second fluid flow path, being output S102.

Further, in some embodiments, the method S100 comprises opening S101 the electronically controllable valve for a first duration of time or until the pressure level, as measured by the pressure sensor, reaches a first pressure value. In some embodiments, the method S100 comprises in response to the pressure change rate being below the pressure change rate value, closing S103 the electronically controllable valve and activating S104 the source of negative pressure for a second duration of time or until the pressure level, as measured by the pressure sensor, reaches a second pressure value.

Executable instructions for performing these functions are, optionally, included in a non-transitory computer-readable storage medium or other computer program product configured for execution by one or more processors of an apparatus for providing negative pressure to a wound dressing.

The herein disclosed technology has been presented above with reference to specific embodiments. However, other embodiments than the above described are possible and within the scope of the claims. Different method steps than those described above, performing the method by hardware or software, may be provided within the scope of the claims. Thus, according to some embodiments embodiment, there is provided a non-transitory computer-readable storage medium storing one or more programs configured to be executed by one or more processors of an apparatus of a negative-pressure wound treatment system, the one or more programs comprising instructions for performing the method according to any one of the above-discussed embodiments.

Generally speaking, a computer-accessible medium may include any tangible or non-transitory storage media or memory media such as electronic, magnetic, or optical media coupled to computer system via bus. The terms "tangible" and "non-transitory," as used herein, are intended to describe a computer-readable storage medium (or "memory") excluding propagating electromagnetic signals, but are not intended to otherwise limit the type of physical computer-readable storage device that is encompassed by the phrase computer-readable medium or memory. For instance, the terms "non-transitory computer-readable medium" or "tangible memory" are intended to encompass types of storage devices that do not necessarily store information permanently, including for example, random access memory (RAM). Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link.

The processor(s) (or control circuitry) 11 (associated with the apparatus 10) may be or include any number of hardware components for conducting data or signal processing or for executing computer code stored in memory. The apparatus 10 may accordingly have an associated memory, and the memory may be one or more devices for storing data and/or computer code for completing or facilitating the various methods described in the present description. The memory may include volatile memory or nonvolatile memory. The memory may include database components, object code components, script components, or any other type of information structure for supporting the various activities of the present description. According to some embodiments, any distributed or local memory device may be utilized with the systems and methods of this description. According to some embodiments the memory is communicably connected to the processor 11 (e.g., via a circuit or any other wired, wireless, or network connection) and includes computer code for executing one or more processes described herein.

It should be noted that any reference signs do not limit the scope of the claims, that some embodiments may be at least in part implemented by means of both hardware and software, and that several "means" or "units" may be represented by the same item of hardware.

Although the figures may show a specific order of method steps, the order of the steps may differ from what is depicted. In addition, two or more steps may be performed concurrently or with partial concurrence. Such variation will depend on the software and hardware systems chosen and on designer choice. All such variations are within the scope of the appended claims. Likewise, software implementations could be accomplished with standard programming techniques with rule-based logic and other logic to accomplish the determining steps, detecting step, generating steps, activating steps, opening steps, closing steps, or outputting steps. The above mentioned and described embodiments are only given as examples and should not be limiting to the appended claims. Other solutions, uses, objectives, and functions within the scope of the below described patent claims should be apparent for the person skilled in the art.

## Claims

1. An apparatus (10) for providing negative pressure to a wound site (27), the apparatus comprising:
a source of negative pressure (14) configured to provide negative pressure via a first fluid flow path from an inlet of the source of negative pressure to a wound site;
an electronically controllable valve (40) configured to release negative pressure from the wound site via a second fluid flow path;
a pressure sensor (15a) configured to measure a pressure level in the second fluid flow path;
control circuitry (11) operatively connected to the source of negative pressure, the electronically controllable valve, and the pressure sensor;
wherein the control circuitry (11) is configured to:
open the electronically controllable valve (40), and
in response to a pressure change rate being above a pressure change rate value, output a signal indicative of a blockage in the second fluid flow path;
in response to the pressure change rate being below the pressure change rate value, close the electronically controllable valve and activate the source of negative pressure (14), and
in response to the pressure level, as measured by the pressure sensor (15a), being non-responsive to a change in negative pressure generated while the source of negative pressure (14) is active, output a signal indicative of a blockage in the first fluid flow path.

2. The apparatus (10) according to claim 1, wherein the control circuitry (11) is configured to operate the apparatus in a pressure regulation mode, a flushing mode, and a blockage-detection mode; and
wherein the control circuitry (11) is configured to:
in the blockage-detection mode:
open the electronically controllable valve (40) at a time after negative pressure has been established at the wound site, and
in response to the pressure change rate being above the pressure change rate value, output the signal indicative of the blockage being present in the second fluid flow path;
in response to the pressure change rate being below the pressure change rate value, close the electronically controllable valve (40) and activate the source of negative pressure (14), and
in response to the pressure level, as measured by the pressure sensor, being non-responsive to the change in negative pressure generated while the source of negative pressure is active, output the signal indicative of the blockage being present in the first fluid flow path.

3. The apparatus (10) according to claim 2, wherein the control circuitry (11) is configured to set the apparatus (10) in the blockage detection mode at a set time after a most recent flushing mode.

4. The apparatus (10) according to any one of claims 1-3, further comprising a user-interface (60) comprising a Light Emitting Diode, LED, (62) arranged to indicate a presence of a blockage to a user of the apparatus, and wherein the control circuitry is further configured to:
activate the LED (62) in response to the signal, indicative of a blockage in the second fluid flow path, being output;
activate the LED (62) in response to the signal, indicative of the blockage in the first fluid flow path, being output.

5. The apparatus (10) according to any one of claims 1-3, further comprising a user-interface (60) comprising a first LED arranged to indicate a presence of a blockage being present in the first fluid flow path to a user of the apparatus and a second LED arranged to indicate a presence of a blockage being present in the second fluid flow path to a user of the apparatus, and wherein the control circuitry is configured to:
activate the second LED in response to the signal, indicative of a blockage in the second fluid flow path, being output;
activate the first LED in response to the signal, indicative of the blockage in the first fluid flow path, being output.

6. The apparatus (10) according to any one of claims 1-5, further comprising a communication interface (56) having at least one antenna and at least one transceiver operatively connected to the at least one antenna; and
wherein the control circuitry (11) is operatively connected to the at least one transceiver and configured to:
transmit a first signal to an external device (50) in response to the signal, indicative of the blockage in the first fluid flow path, being output, and
transmit a second signal to the external device (50) in response to the signal, indicative of a blockage in the second fluid flow path, being output.

7. The apparatus (10) according to any one of claims 1-6, wherein the control circuitry (11) is configured to open the electronically controllable valve (40) for a first duration of time or until the pressure level, as measured by the pressure sensor (15a), reaches a first pressure value.

8. The apparatus (10) according to any one of claims 1-7, wherein the control circuitry (11) is configured to:
in response to the pressure change rate being below the pressure change rate value, close the electronically controllable valve (40) and activate the source of negative pressure (14) for a second duration of time or until the pressure level, as measured by the pressure sensor (15a), reaches a second pressure value.

9. A method (S100) for operating an apparatus for providing negative pressure to a wound site, wherein the apparatus comprises a source of negative pressure configured to provide negative pressure via a first fluid flow path from an inlet of the source of negative pressure to a wound site, an electronically controllable valve configured to release negative pressure from the wound site via a second fluid flow path, and a pressure sensor configured to measure a pressure level in the second fluid flow path, the method comprising:
opening (S101) the electronically controllable valve, and
in response to a pressure change rate being above a pressure change rate value, outputting (S102) a signal indicative of a blockage in the second fluid flow path;
in response to the pressure change rate being below the pressure change rate value, closing (S103) the electronically controllable valve and activating (S104) the source of negative pressure, and
in response to the pressure level, as measured by the pressure sensor, being non-responsive to a change in negative pressure generated while the source of negative pressure is active, outputting (S105) a signal indicative of a blockage in the first fluid flow path.

10. The method (S100) according to claim 9, wherein the apparatus is configured to operate in a pressure regulation mode, a flushing mode, and a blockage-detection mode, and wherein the method further comprises:
during the blockage-detection mode:
opening (S101) the electronically controllable valve at a time after negative pressure has been established at the wound site, and
in response to the pressure change rate being above the pressure change rate value, outputting (S102) the signal indicative of the blockage being present in the second fluid flow path;
in response to the pressure change rate being below the pressure change rate value, closing (S103) the electronically controllable valve and activating (S104) the source of negative pressure, and
in response to the pressure level, as measured by the pressure sensor, being non-responsive to the change in negative pressure generated while the source of negative pressure is active, outputting (S105) the signal indicative of the blockage being present in the first fluid flow path.

11. The method (S101) according to any one of claims 9-10, wherein the apparatus further comprises a user-interface comprising a Light Emitting Diode, LED, arranged to indicate a presence of a blockage to a user of the apparatus;
wherein outputting (S102) the signal indicative of the blockage being present in the second fluid flow path comprises activating the LED; and
wherein outputting (S105) the signal indicative of the blockage being present in the first fluid flow path comprises activating the LED.

12. The method (S101) according to any one of claims 9-10, wherein the apparatus further comprises a user-interface comprising a first LED arranged to indicate a presence of a blockage being present in the first fluid flow path to a user of the apparatus and a second LED arranged to indicate a presence of a blockage being present in the second fluid flow path to a user of the apparatus:
wherein outputting (S102) the signal indicative of the blockage being present in the second fluid flow path comprises activating the second LED;
wherein outputting (S105) the signal indicative of the blockage being present in the first fluid flow path comprises activating the first LED.

13. A computer program product comprising instructions which, when the program is executed by a computing device of an apparatus for providing negative pressure to a wound site, causes the apparatus to carry out the method (S101) according to any one of claims 9-12.

14. A computer-readable storage medium comprising instructions which, when executed by a computing device of an apparatus for providing negative pressure to a wound site, causes the apparatus to carry out the method (S101) according to any one of claims 9-12.

15. A system (1) comprising:
an apparatus (10) according to any one of claims 1-8;
a wound cover (22) for creating a sealed space defined in part by the wound site (27); and
a tubing assembly defining the first fluid flow path and the second fluid flow path.
